# EUROPÄISCHE PATENTSCHRIFT

(11) **EP 1 658 013 B1**
(45) Veröffentlichungstag und Bekanntmachung des Hinweises auf die Patenterteilung: **31.12.2008**
(21) Anmeldenummer: 03818372.9
(22) Anmeldetag: 29.08.2003
(51) Int. Cl.: A61B 17/72

(54) **MARKNAGEL**
INTRAMEDULLARY NAIL
CLOU INTRAMEDULLAIRE

(43) Veröffentlichungstag der Anmeldung: 24.05.2006
(73) Patentinhaber: Synthes GmbH, 4436 Oberdorf (CH)
(72) Erfinder: FRIGG, Robert, CH-2544 Bettlach (CH); FUHRER, Marcel, 3036 Detligen (CH)
(74) Vertreter: Lusuardi, Werther
(86) Internationale Anmeldenummer: PCT/CH2003/000591
(87) Internationale Veröffentlichungsnummer: WO 2005/020830

(56) Entgegenhaltungen:
- EP-A- 1 053 718
- WO-A-02/060331
- US-A- 5 041 115
- US-A- 5 658 287
- US-A- 6 123 708
- US-B1- 6 454 810

## Beschreibung

Die Erfindung bezieht sich auf einen Marknagel gemäss dem Oberbegriff des Patentanspruchs 1.

Das Dokument US-A-6 123 708, von dem der Oberbegriff des Anspruchs 1 abgeleitet wird, offenbart einen gattungsgemäßen Marknagel. Dieser weist quer zu seiner Längsachse Bohrungen zur Aufnahme von Verriegelungselementen sowie einen parallel zu dieser Längsachse verlaufenden Längsschlitz auf.

Aus der CH-A5 668 173 KLAUE ist ein weiterer Marknagel bekannt, der an seinem distalen Ende einen Längsschlitz aufweist. Dieser bekannte Marknagel ist dazu bestimmt erst nach erfolgter Implantation des dazugehörenden Verriegelungselementes (einer Schraube oder einem Bolzen) in den Markraum eingeführt zu werden und trifft dann mit seiner geschlitzten Spitze auf die Verriegelungsschraube, wodurch sich der Schlitz aufweitet, so dass der--Marknagel über die Verriegelungschraube bis in seine Endposition gleiten kann. Die zuerst gesetzte Verriegelungsschraube dient somit als Zielhilfe für den später zu implantierenden Marknagel. Um für diesen Zweck geeignet zu sein, ist der Längsschlitz dieses bekannten Marknagels im Vergleich zum Durchmesser der Verriegelungsschraube relativ breit, da sich sonst der Längsschlitz nicht öffnen könnte. Der relativ breite Längsschlitz weist aber zwei Nachteile auf: Erstens wird die Spitze des Marknagels in seiner Festigkeit stark geschwächt und zweitens ist es jederzeit wieder möglich, dass sich der Marknagel gegenüber der Verriegelungschraube axial verschiebt.

Hier will die Erfindung Abhilfe schaffen. Der Erfindung liegt die Aufgabe zugrunde, einen geschlitzten Marknagel zu schaffen, der nach Einführung des Verriegelungselementes keine gegenseitige axiale Verschiebbarkeit mehr zulässt.

Die Erfindung löst die gestellte Aufgabe mit einem Marknagel, welcher die Merkmale des Anspruchs 1 aufweist.

Die Vorteile des erfindungsgemässen Marknagels sind vielfältig:
a) Die reduzierte Nagelsteifigkeit vereinfacht die Implantation;
b) Bei der Verwendung von Verriegelungsbolzen, deren Durchmesser leicht grösser ist als die Querbohrung im Marknagel, kann der Bolzen durch elastische Deformation des Nagels verklemmt werden, was zu einer besseren Verankerung des Nagels im Knochen führt;
c) Bei kleinen Nageldurchmessern können durch die Elastizität im Schlitzbereich, wo sich auch die Verriegelungsbohrungen befinden, im Durchmesser grössere Verriegelungsbolzen verwendet werden (bei herkömmlichen Nägeln würde dies zu einer Schwächung des Nagelquerschnitts, durch zu grosse Bohrungen führen).
d) Durch die schlitzbedingte Elastizität im Bereich der Verriegelungsbohrungen können schädliche Stresskonzentrationen verringert werden
e) Es ist keine relative Längsverschiebung zwischen Marknagel und Verriegelungsschraube möglich, ohne dass eine plastische Deformation des Marknagels oder der Verriegelungselemente erfolgt; und
f) der oder die Verriegelungselemente werden geklemmt und spielfrei gegen Abwinklungen und jegliches Verschieben gesichert.

Bei einer besonderen Ausführungsform entspricht die Schlitzbreite "b" des Marknagels höchstens dem 0,5-fachen, vorzugsweise höchstens dem 0,4-fachen des kleinsten der definierten Durchmesser der Bohrungen. Dadurch ist der Marknagel in optimaler Weise flexibel beim Einbringen und die Verriegelungselemente werden optimal winkelstabil fixiert, bzw. geklemmt.

Bei einer weiteren Ausführungsform beträgt die Anzahl der Bohrungen (10,11) zwei, wobei der Durchmesser "d10" der näher an der Öffnung des Längsschlitzes liegenden Bohrung kleiner ist als der Durchmesser "d11" der anderen Bohrung. Dadurch können grösser dimensionierte Verriegelungselemente verwendet werden, wodurch sich weniger Bolzenbrüche ergeben.

Der Längsschlitz des Marknagels weist eine Länge L auf, welche vorzugsweise mindestens dem 10-fachen, typischerweise dem 15-fachen des kleinsten der definierten Durchmesser "d10" der Bohrungen entspricht. Dadurch ist der Marknagel beim Einbringen flexibel.

Die Schlitzbreite "b" sollte vorzugsweise im wesentlichen über die gesamte Schlitzlänge L konstant sein. Dadurch ergibt sich eine herstellungstechnische Vereinfachung sowie eine minimale Schwächung des Marknagels bei kleinstmöglicher Schlitzbreite.

Bei einer besonderen Ausführungsform beginnt der Längsschlitz am proximalen Ende des Marknagels. Gegenüber einem Marknagel mit üblicherweise distal angebrachtem Schlitz besitzt ein Marknagel mit proximal angeordnetem Schlitz den Vorteil, dass er mittels eines geeigneten Einschlag-Instrumentariums elastisch vorgespreizt werden kann, um sodann die Verriegelungselemente durch den Zielbügel des Instrumentariums in den Marknagel einzubringen. Nach dem Entfernen des Instrumentariums schliesst sich der proximale Schlitz dann wieder, wodurch die Verriegelungselemente im Marknagel festgeklemmt werden.

Bei einer weiteren Ausführungsform mündet der Längsschlitz weder am distalen Ende noch am proximalen Ende des Marknagels. Diese geschlossene Version hat den Vorteil, dass ein ungewollt grosses Aufspreizen des distalen (oder proximalen) Endes des Marknagels - speziell bei Marknägeln mit kleinem Durchmesser - verhindert wird. Eine solche Aufspreizung kann zu einer Stresskonzentration am Schlitzende führen. Ist der Schlitz beidseitig geschlossen, können die Verriegelungselemente zudem stärker festgeklemmt werden.

Der Marknagel kann in der Richtung der Längsachse auch hohl ausgebildet sein.

Die in Bohrungen des Marknagels einzuführenden Verriegelungselemente weisen einem definierten Durchmesser auf, welche vorzugsweise grösser ist als der definierte Durchmesser der dazugehörenden Bohrung. Der definierte Durchmesser des Verriegelungselementes kann aber gleich gross sein wie der definierte Durchmesser der dazugehörenden Bohrung. Die letztere Ausführung hat den Vorteil einer reduzierten Nagelsteifigkeit und einer vereinfachen Implantation.

Der definierte Durchmesser des Verriegelungselementes kann aber auch mindestens das 1,1-fache, vorzugsweise das 1,2-fache des definierten Durchmessers der dazugehörenden Bohrung betragen. Bei dieser Ausführung können bei gleichbleibendem Nagelquerschnitt grössere Verriegelungselemente verwendet werden und es lassen sich damit bei kleinen Marknägeln Brüche der Verriegelungselemente verhindern.

Der Durchmesser des näher an der Öffnung des Längsschlitzes eingeführten Verriegelungselementes ist vorzugsweise grösser als der Durchmesser der übrigen Verriegelungselemente.

Durch das Einführen des Verriegelungselementes in der Bohrung ist der Marknagel im Bereich des Längsschlitzes elastisch gespreizt. Die Länge L des Längsschlitzes sollte vorzugsweise derart gewählt werden, dass sich der Marknagel beim Einsetzen der Verriegelungselemente nur im elastischen Bereich deformiert.

Bei einer weiteren Ausführungsform weist der Marknagel eine zusätzliche, senkrecht zur Ebene der Bohrungen verlaufende Verriegelungsbohrung auf. Dies führt zu einer Erhöhung- der Nagelsteifigkeit nach erfolgter Implantation und nach dem Setzen der Verriegelungselemente in der Ebene der beiden Bohrungen. Die Nagelelastizität wird durch den Längsschlitz erreicht und vereinfacht die Nagelimplantation. Wenn der Nagel aber einmal implantiert ist, ist die Wiederversteifung speziell bei dünnen Nägeln wünschenswert.

Weitere vorteilhafte Ausführungen sind in den abhängigen Ansprüchen enthalten.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen teilweisen Längsschnitt durch einen im distalen Teil offen geschlitzten bekannten Marknagel;
Fig. 2 einen teilweisen Längsschnitt nach Fig. 1 mit eingesetzten Verriegelungselementen;
Fig. 3 einen teilweisen Längsschnitt nach Fig. 2 um 90° verdreht;
Fig. 4 einen teilweisen Längsschnitt nach Fig. 2 mit einer zusätzlichen Querbohrung;
Fig. 5 einen vergrösserten Ausschnitt aus Fig. 4 im Bereich der zusätzlichen Querbohrung;
Fig. 6 einen teilweisen Längsschnitt durch einen im proximalen Teil offen geschlitzten bekannten Marknagel mit aufgesetztem Zielbügel;
Fig. 7 einen teilweisen Längsschnitt nach Fig. 6 um 90° verdreht;
Fig. 8 einen teilweisen Längsschnitt nach Fig. 7 mit einem eingesetzten Verriegelungselement und abgenommenem Zielbügel;
Fig. 9 einen teilweisen-Längsschnitt durch einen erfindungsgemäßen Marknagel, der im distalen Teil einen ungeöffneten Schlitz aufweist;
Fig. 10 einen teilweisen Längsschnitt nach Fig. 9 mit eingesetzten Verriegelungselementen; und
Fig. 11 einen teilweisen Längsschnitt durch einen im distalen Teil offen geschlitzten erfindungsgemäßen Marknagel mit einer Schlitzsicherung.

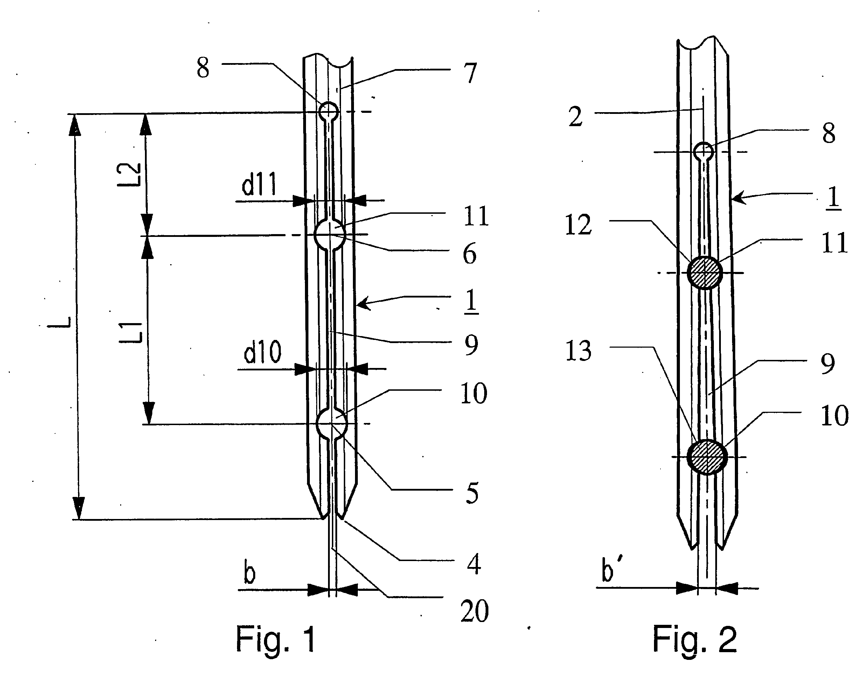

Der in Fig. 1 dargestellte distale Teil des Marknagels 1 besitzt eine Längsachse 2, ein proximales Ende 3, ein distales Ende 4, sowie zwei quer zur Längsachse 2 verlaufende, senkrecht zur Zeichenebene liegende Bohrungen 10,11 mit den Durchmessern d10 und d11 zur Aufnahme von Verriegelungselementen 12,13 (Fig. 2) in Form von Verriegelungsschrauben, sowie einen parallel zur Längsachse 2 senkrecht zur Zeichenebene verlaufenden, am distalen Ende 4 beginnenden Längsschlitz 9 konstanter Breite b und mit der Länge L = (20 x "d10"). Der Durchmesser "d10" der näher am offenen Ende des Längsschlitzes 9 liegenden Bohrung 10 ist dabei etwas grösser als der Durchmesser "d11" der anderen Bohrung 11.
Im Bereich zwischen den beiden Bohrungen 10,11 weist der Längsschlitz 9 eine - in der Zeichenebene gemessene - Schlitzbreite b = (0,2 x "d10") auf.

Die beiden Bohrungen 10,11 weisen je ein Zentrum 5,6 auf. Der Längsschlitz 9 erstreckt sich von seiner am distalen Ende 4 gelegenen Öffnung über die beiden Bohrungen (10,11) noch ein Stück weiter nach proximal bis zum Schlitzgrund 8 in Form einer kleinen Bohrung mit sehr kleinem Durchmesser. Der Abstand zwischen dem Schlitzgrund 8 und dem Zentrum 6 der Bohrung 11 beträgt "L2". Je grösser "L2" ist desto flexibler ist der Marknagel.
Der Abstand zwischen den beiden Zentren 5,6 der beiden -Bohrungen 10,11 beträgt "L1". In dieser Ausführungsform beträgt "L1" ungefähr 30 mm.
Im weiteren weist der Marknagel 1 eine durchgehende, koaxial zur Längsachse 2 verlaufende Kannulierung 7 auf.

Wie in Fig. 2 dargestellt können durch die beiden Bohrungen 10,11 zwei Verriegelungselemente 12,13 in Form- von Knochenschrauben -eingeführt werden. Dabei erfolgt eine Aufspreizung des Längsschlitzes 9, so dass sich dessen Breite am offenen Ende des Längsschlitzes vom Betrag "b" zum Betrag b'> b vergrössert.

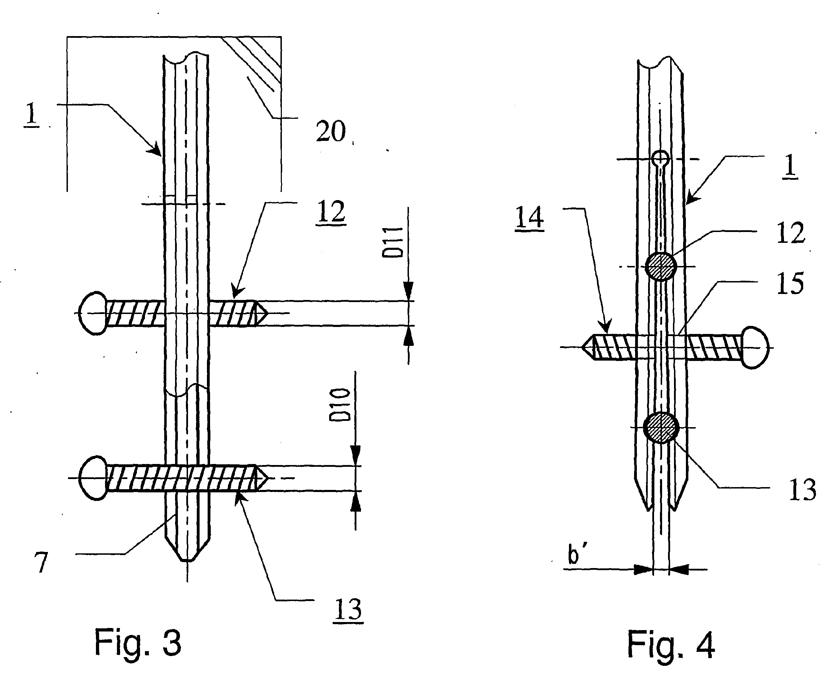

Wie in Fig. 3 dargestellt weist der Schaft der beiden Verriegelungselemente 12,13 einem Durchmesser D10, D11 auf welche dem 1,2-fachen des Durchmessers der entsprechenden Bohrung 10,11 entspricht, so dass nach Einführung der beiden Verriegelungselemente 12,13 in die Bohrungen 10,11 der Marknagel 1 im Bereich seines Längsschlitzes 9 elastisch gespreizt ist, wie dies in Fig. 4 dargestellt ist.

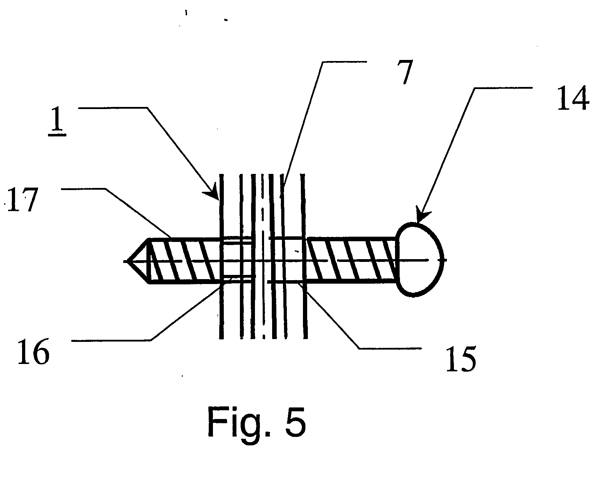

In Fig. 4 ist eine Variante des Marknagels 1 dargestellt, bei welcher noch eine dritte Bohrung 15 zwischen den beiden Bohrung 10,11 angeordnet ist. Die Bohrung 15 ist Relativ zu den beiden anderen Bohrung 10,11 um 90° verdreht. Wie in den Fig. 4 und 5 dargestellt kann in diese zusätzliche Bohrung 15 ein zusätzliches Verriegelungselement 14 in Form einer Verriegelungsschraube eingeführt werden. Im eintrittsfernen Segment des Marknagels 1 ist ein Innengewinde 16 vorgesehen, in welches das Aussengewinde 17 des Verriegelungselementes 14 greift, so dass der Marknagel 1 im geschlitzten Bereich - durch das Verriegelungselement 14 - wieder zusammengehalten wird.

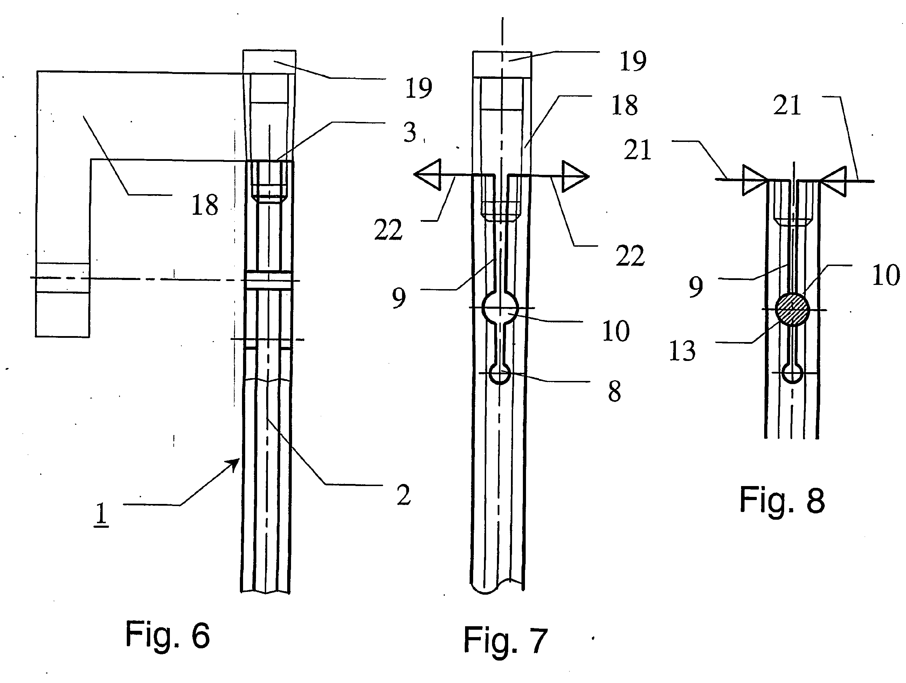

In den Fig. 6 - 8 ist eine Variante des Marknagels 1 dargestellt, bei welcher der Längsschlitz 9 nicht am distalen Ende 4, sondern am proximalen Ende 3 des Marknagels 1 angebracht ist. Bei diesem Ausführungsbeispiel ist nur eine einzige Bohrung 10 vorgesehen zur Aufnahme eines einzigen Verriegelungselementes 13 in Form einer Verriegelungsschraube. Ansonsten entspricht diese Ausführung der Ausführung gemäss den Figuren 1 und 2 für einen distal geschlitzten Marknagel 1. Wie in Fig. 6 dargestellt erfolgt das Einsetzen des Verriegelungselementes 13 in die Bohrung 10 bei aufgesetztem Zielbügel 18; dabei wird das geschlitzte proximale Ende des Marknagels 1 durch die Verbindungsschraube 19 - wie durch die Pfeile 22 (Fig. 7) angedeutet - aufgespreizt, so dass das gegenüber der Bohrung 10 überdimensionierte Verriegelungselemente 13 problemlos in diese eingesetzt werden kann. Anschliessend wird die Verbindungsschraube 19 wieder gelöst, so dass der Zielbügel 18 entfernt werden kann. Dabei versucht sich der geschlitzte Marknagel 1 am proximalen Ende 3 - wie durch die Pfeile 21 (Fig. 8) angedeutet - wieder zusammenzuziehen und fixiert --damit das eingeführte Verriegelungselement 13 winkelstabil in der Bohrung 10.

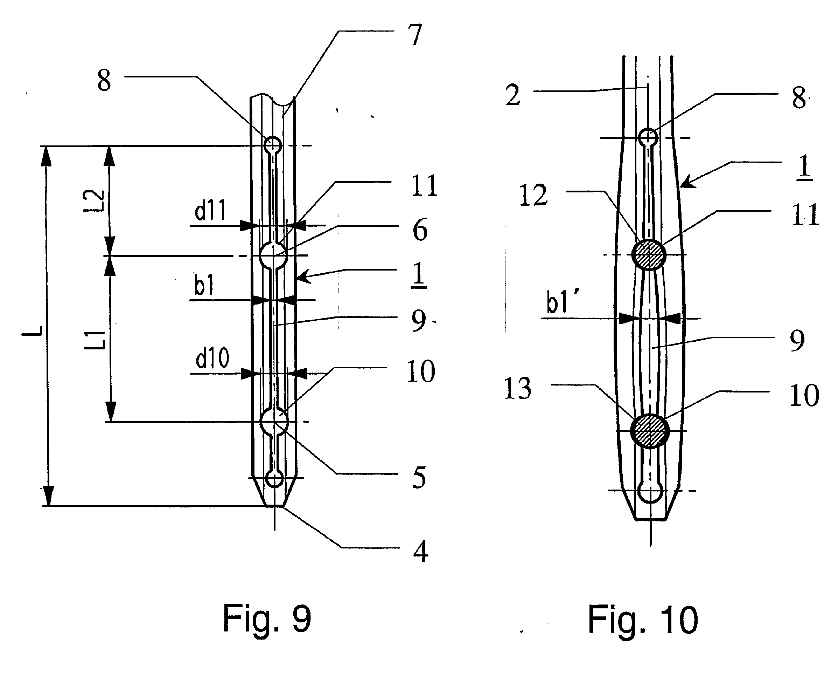

In den Fig. 9 und 10 ist eine weitere Variante des Marknagels 1 dargestellt, bei welcher der distal angeordnete Schlitz 9 an seinen beiden Enden geschlossen ist, d.h. sich nicht wie bei der Ausführung gemäss den Fig. 1 - 4 am distalen Ende 4 des Marknagels 1 geöffnet ist. Durch diese Bauweise wird beim Einführen der beiden Verriegelungselemente 12 und 13 in die Bohrungen 10 und 11 der Längsschlitz 9 von seiner ursprünglichen Breite b1 auf eine Breite b1' aufgespreizt. Der aufgespreizte Längsschlitz 9 übt somit eine dauernde Klemmkraft auf die beiden Verriegelungselemente 12 und 13 in den Bohrungen 10 und 11 aus, so dass diese sicher darin festgehalten werden. Je länger L ist , desto flexibler ist der Marknagel und desto einfacher ist dessen Insertion. "L1" ist individuell anpassbar und hat keinen Einfluss auf die Funktion. Umso länger "L2" ist, desto einfacher ist das Einbringen der Verriegelungselemente 12, und 13 und desto weniger besitzen letztere Winkelstabilität. Ansonsten entspricht diese Ausführung der Ausführungsform gemäss den Figuren 1 - 4.

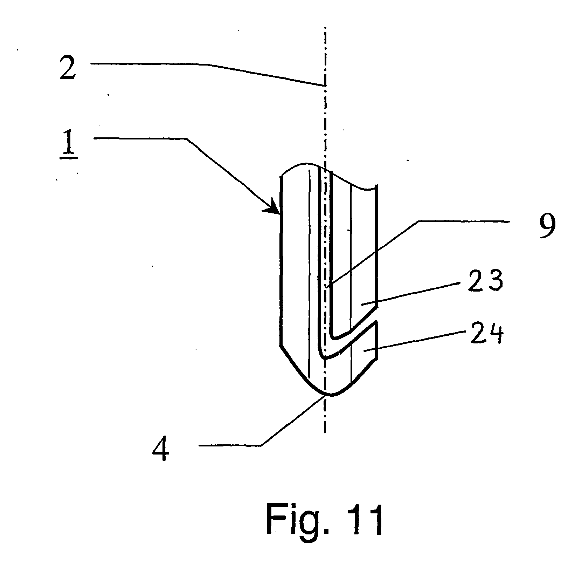

In Fig. 11 ist eine weitere Variante des Marknagels 1 dargestellt, bei welcher der distal angeordnete Schlitz 9 nicht durchgehend geradlinig, d.h. koaxial zur Längsachse 2 verläuft, sondern kurz vor dem distalen Ende 4 abgebogen ist, so dass der Schlitz 9 lateral (hier auf der rechten Seite) mündet. Durch die Umlenkung des Schlitzes 9 wird eine Sicherung 23,24 in der Art eines "Schwalbenschwanzes" realisiert, welche die Spreizbarkeit des Schlitzes 9 limitiert. Der rechte Teil 23 des Marknagels 1 kommt bei einer Spreizung des Schlitzes 9 mit seinem angeschrägten Ende gegen das korrespondierende angeschrägte Ende 24 des linken, abgebogenen Teil 24 des Marknagels 1 zur Anlage und verhindert dadurch eine weitere, übermässige Aufspreizung des Marknagels im seinem distalen Teil.

## Patentansprüche

1. Marknagel (1) mit einer Längsachse (2), einem proximalen Ende (3), einem distalen Ende (4), mindestens einer quer zur Längsachse (2) verlaufenden, in einer Ebene (20) liegenden Bohrung (10;11) mit einem definierten Durchmesser ("d10";"d11") zur Aufnahme eines Verriegelungselementes (12,13), sowie einem parallel zur Längsachse (2) in der Ebene (20) verlaufenden Längsschlitz (9), wobei der Längsschlitz (9) im ungespreizten Zustand des Marknagels (1) eine senkrecht zur Ebene (20) im Bereich ausserhalb der Bohrungen (10,11) gemessene Schlitzbreite "b" aufweist, welche höchstens dem 0,6-fachen des kleinsten der definierten Durchmesser ("d10") der Bohrungen (10,11) entspricht,
**dadurch gekennzeichnet, dass** der Längsschlitz (9) entweder an den Enden (3,4) geschlossen ist oder eine Sicherung (23,24) aufweist, so dass die Spreizung des Längsschlitzes (9) betragsmässig begrenzt ist.

2. Marknagel (1) nach Anspruch 1, **dadurch gekennzeichnet, dass** die Schlitzbreite "b" höchstens dem 0,5-fachen, vorzugsweise höchstens dem 0,4-fachen des kleinsten der definierten Durchmesser ("d10") der Bohrungen (10,11) entspricht.

3. Marknagel (1) nach Anspruch 1 oder 2, **dadurch gekennzeichnet, dass** die Anzahl der Bohrungen (10,11) zwei beträgt, wobei der Durchmesser "d10" der näher an der Öffnung des Längsschlitzes (9) liegenden Bohrung (10) kleiner ist als der Durchmesser "d11" der anderen Bohrung (11).

4. Marknagel (1) nach einem der Ansprüche 1 bis 3, **dadurch gekennzeichnet, dass** der Längsschlitz (9) eine Länge L aufweist, welche mindestens dem 10-fachen des kleinsten der definierten Durchmesser (d10) der Bohrungen (10,11) entspricht.

5. Marknagel (1) nach Anspruch 4, **dadurch gekennzeichnet, dass** der Längsschlitz (9) eine Länge L aufweist, welche mindestens dem 15-fachen des kleinsten der definierten Durchmesser (d10) der Bohrungen (10,11) entspricht.

6. Marknagel (1) nach einem der Ansprüche 1 bis 5, **dadurch gekennzeichnet, dass** die Schlitzbreite b im wesentlichen über die gesamte Schlitzlänge L konstant ist.

7. Marknagel (1) nach einem der Ansprüche 1 bis 6, **dadurch gekennzeichnet, dass** der Längsschlitz (9) am proximalen Ende (3) des Marknagels (1) beginnt.

8. Marknagel (1) nach einem der Ansprüche 1 bis 7, **dadurch gekennzeichnet, dass** der Längsschlitz (9) weder am distalen Ende (4) noch am proximalen Ende (3) mündet.

9. Marknagel (1) nach einem der Ansprüche 1 bis 8, **dadurch gekennzeichnet, dass** er in der Richtung der Längsachse (2) hohl ausgebildet ist.

10. Marknagel (1) nach einem der Ansprüche 1 bis 9, **dadurch gekennzeichnet dass** es mindestens ein Verriegelungselement (12,13,14) mit einem definierten Durchmesser (D10, D11) aufweist.

11. Marknagel (1) nach Anspruch 10, **dadurch gekennzeichnet dass** der definierte Durchmesser (D10, D11) des Verriegelungselements (12,13) grösser ist als der definierte Durchmesser (d10,d11) der dazugehörenden Bohrung (10,11).

12. Marknagel (1) nach Anspruch 10, **dadurch gekennzeichnet dass** der definierte Durchmesser (D10, D11) des Verriegelungselement (12,13) gleich gross ist wie der definierte Durchmesser (d1 0, d11) der dazugehörenden Bohrung (10,11).

13. Marknagel (1) nach Anspruch 10, **dadurch gekennzeichnet dass** der definierte Durchmesser (D10, D11) des Verriegelungselementes (12,13) mindestens das 1,1-fache, vorzugsweise das 1,2-fache des definierten Durchmessers (d10, d11) der dazugehörenden Bohrung (10,11) beträgt.

14. Marknagel (1) nach einem der Ansprüche 10 bis 13, **dadurch gekennzeichnet dass** der Durchmesser des näher an der Öffnung des Längsschlitzes (9) eingeführten Verriegelungselementes (13) grösser ist als der Durchmesser der übrigen Verriegelungselemente (12).

15. Marknagel (1) nach einem der Ansprüche 10 bis 14, **dadurch gekennzeichnet dass** das Verriegelungselement (12,13) in der Bohrung (10) eingeführt ist und der Marknagel (1) im Bereich des Längsschlitzes (9) elastisch gespreizt ist.

16. Marknagel (1) nach einem der Ansprüche 1 bis 15, **dadurch gekennzeichnet dass** die Länge L des Längsschlitzes (9) derart gewählt wird, dass sich der Marknagel (1), beim Einsetzen der Verriegelungselemente (12,13), nur im elastischen Bereich deformiert.

17. Marknagel (1) nach einem der Ansprüche 1 bis 16, **dadurch gekennzeichnet dass** er eine zusätzliche, senkrecht zur Ebene (7) verlaufende Verriegelungsbohrung (15) aufweist.

## Claims

1. An intramedullary pin (1) with a longitudinal axis (2), a proximal end (3), a distal end (4), at least one hole (10, 11) that extends transversely to the longitudinal axis (2) and situated in a plane (20), having a defined diameter (d10, d11) to accommodate a locking element (12, 13), as well as a longitudinal slot (9) extending in the plane (20) parallel to the longitudinal axis (2), whereby in the unexpanded state of the intramedullary pin (1) the longitudinal slot (9) has a width b, measured perpendicularly to the plane (20) in the region outside of the holes (10, 11), said width b being maximum 0.6 times that of the smallest defined diameter (d10) of the holes (10, 11),
**characterised in that** the slot (9) is either closed at the ends (3,4) or has a safety device (23, 24) so that the expansion of the slot (9) is limited with regard to its amount.

2. An intramedullary pin (1) according to claim 1, **characterised in that** the width b of the slot is maximum 0.5 times, preferably maximum 0.4 times that of the smallest defined diameter (d10) of the holes (10, 11).

3. An intramedullary pin (1) according to claim 1 or 2, **characterised in that** the number of holes (10, 11) is two, while the diameter d10 of the hole closer to the opening of the longitudinal slot (9) is smaller than diameter d11 of the other hole (11).

4. An intramedullary pin (1) according to any one of claims 1 to 3, **characterised in that** the longitudinal slot (6) has a length of L, that is at least 10 times that of the smallest defined diameter (d10) of the holes (10, 11).

5. An intramedullary pin (1) according to any one of claims 1 to 4, **characterised in that** the longitudinal slot (6) has a length of L, that is at least 15 times that of the smallest defined diameter (d10) of the holes (10, 11).

6. An intramedullary pin (1) according to any one of claims 1 to 5, **characterised in that** the width b of the slot is essentially constant over the entire length L of the slot.

7. An intramedullary pin (1) according to any one of claims 1 to 6, **characterised in that** the longitudinal slot (9) commences at the proximal end (3) of the intramedullary pin (1).

8. An intramedullary pin (1) according to any one of claims 1 to 7, **characterised in that** the longitudinal slot (9) terminates neither at the distal end (4) nor at the proximal end (3).

9. An intramedullary pin (1) according to any one of claims 1 to 8, **characterised in that** it has a hollow construction in the direction of the longitudinal axis (2).

10. An intramedullary pin (1) according to any one of claims 1 to 9, **characterised in that** it has at least one locking element (12, 13, 14) with a defined diameter (D10, D11).

11. An intramedullary pin (1) according to claim 10, **characterised in that** the defined diameter (D10, D11) of the locking element (12, 13) is larger than the defined diameter (d 10, d11) of the associated hole (10, 11).

12. An intramedullary pin (1) according to claim 10, **characterised in that** the defined diameter (D10, D11) of the locking element (12, 13) is the same as the defined diameter (d10, d11) of the associated hole (10, 11).

13. An intramedullary pin (1) according to claim 10, **characterised in that** the defined diameter (D10, D11) of the locking element (12, 13) is at least 1.1 times, preferably 1.2 times that of the defined diameter (d 10, d11) of the associated hole (10, 11).

14. An intramedullary pin (1) according to any one of claims 10 to 13, **characterised in that** the diameter of the locking element (13), introduced closer to the opening of the longitudinal slot (9) is larger than the diameter of the other locking elements (12).

15. An intramedullary pin (1) according to any one of claims 10 to 14, **characterised in that** the locking element (12, 13) is introduced into the hole (10) and the intramedullary pin (1) is elastically expanded in the region of the longitudinal slot (9).

16. An intramedullary pin (1) according to any one of claims 1 to 15, **characterised in that** the length L of the longitudinal slot (9) is so chosen, that during the insertion of the locking elements (12, 13) the intramedullary pin (1) deforms only in the elastic range.

17. An intramedullary pin (1) according to any one of claims 1 to 16, **characterised in that** it has an additional locking hole (15), extending at right angle to the plane (7).

## Revendications

1. Clou intramédullaire (1) présentant un axe longitudinal (2), une extrémité proximale (3), une extrémité distale (4), au moins un alésage (10 ; 11) ayant un diamètre défini ("d10" ; "d11"), situé dans un plan (20) s'étendant transversalement à l'axe longitudinal (2), destiné à loger un élément de verrouillage (12, 13), ainsi qu'une fente longitudinale (9) s'étendant dans le plan (20) parallèlement à l'axe longitudinal (2), dans lequel la fente longitudinale (9) présente, à l'état non écarté du clou intramédullaire (1), une largeur de fente "b" mesurée perpendiculairement au plan (20) dans la zone située hors des alésages (10, 11), largeur qui correspond au maximum à 0,6 fois la valeur du plus petit des diamètres définis ("d10") des alésages (10, 11),
**caractérisé en ce que** la fente longitudinale (9) est fermée au niveau des extrémités (3, 4) ou bien présente un dispositif de blocage (23, 24) destiné à limiter la valeur d'écartement de la fente longitudinale (9).

2. Clou intramédullaire (1) selon la revendication 1, **caractérisé en ce que** la largeur de fente "b" correspond au maximum à 0,5 fois, de préférence au maximum à 0,4 fois la valeur du plus petit des diamètres définis ("d10") des alésages (10, 11).

3. Clou intramédullaire (1) selon la revendication 1 ou 2, **caractérisé en ce que** le nombre des alésages (10, 11) est de deux, le diamètre "d10" de l'alésage (10) situé plus près de l'ouverture de la fente longitudinale (9) étant plus petit que le diamètre "d11" de l'autre alésage (11).

4. Clou intramédullaire (1) selon l'une quelconque des revendications 1 à 3, **caractérisé en ce que** la fente longitudinale (9) présente une longueur L, qui correspond au moins à 10 fois la valeur du plus petit des diamètres définis (d10) des alésages (10, 11).

5. Clou intramédullaire (1) selon la revendication 4, **caractérisé en ce que** la fente longitudinale (9) présente une longueur L, qui correspond au moins à 15 fois la valeur du plus petit des diamètres définis (d10) des alésages (10, 11).

6. Clou intramédullaire (1) selon l'une quelconque des revendications 1 à 5, **caractérisé en ce que** la largeur de fente b est essentiellement constante sur toute la longueur L de la fente.

7. Clou intramédullaire (1) selon l'une quelconque des revendications 1 à 6, **caractérisé en ce que** la fente longitudinale (9) commence au niveau de l'extrémité proximale (3) du clou intramédullaire (1).

8. Clou intramédullaire (1) selon l'une quelconque des revendications 1 à 7, **caractérisé en ce que** la fente longitudinale (9) ne débouche ni au niveau de l'extrémité distale (4), ni au niveau de l'extrémité proximale (3).

9. Clou intramédullaire (1) selon l'une quelconque des revendications 1 à 8, **caractérisé en ce qu'**il est réalisé sous une forme creuse dans le sens de l'axe longitudinal (2).

10. Clou intramédullaire (1) selon l'une quelconque des revendications 1 à 9, **caractérisé en ce qu'**il présente au moins un élément de verrouillage (12, 13, 14) ayant un diamètre défini (D10, D11).

11. Clou intramédullaire (1) selon la revendication 10, **caractérisé en ce que** le diamètre défini (D10, D11) de l'élément de verrouillage (12, 13) est plus grand que le diamètre défini (d10, d11) de l'alésage correspondant (10, 11).

12. Clou intramédullaire (1) selon la revendication 10, **caractérisé en ce que** le diamètre défini (D10, D11) de l'élément de verrouillage (12, 13) est de la même dimension que le diamètre défini (d10, d11) de l'alésage correspondant (10, 11).

13. Clou intramédullaire (1) selon la revendication 10, **caractérisé en ce que** le diamètre défini (D10, D11) de l'élément de verrouillage (12, 13) correspond au moins à 1,1 fois, de préférence à 1,2 fois la valeur du diamètre défini (d10, d11) de l'alésage correspondant (10, 11).

14. Clou intramédullaire (1) selon l'une quelconque des revendications 10 à 13, **caractérisé en ce que** le diamètre de l'élément de verrouillage (13) introduit plus près de l'ouverture de la fente longitudinale (9) est plus grand que le diamètre des autres éléments de verrouillage (12).

15. Clou intramédullaire (1) selon l'une quelconque des revendications 10 à 14, **caractérisé en ce que** l'élément de verrouillage (12, 13) est introduit dans l'alésage (10) et le clou intramédullaire (1) est écarté de manière élastique dans la zone de la fente longitudinale (9).

16. Clou intramédullaire (1) selon l'une quelconque des revendications 1 à 15, **caractérisé en ce que** la longueur L de la fente longitudinale (9) est choisie de telle manière que lorsque les éléments de verrouillage (12, 13) sont insérés, le clou intramédullaire (1) se déforme uniquement dans la zone élastique.

17. Clou intramédullaire (1) selon l'une quelconque des revendications 1 à 16, **caractérisé en ce qu'**il présente un alésage de verrouillage (15) supplémentaire s'étendant perpendiculairement au plan (7).
